(19) Europäisches Patentamt
European Patent Office
Office européen des brevets

(11) Veröffentlichungsnummer: **0 000 471**
**A1**

(12) # EUROPÄISCHE PATENTANMELDUNG

(21) Anmeldenummer: **78100135.9**

(22) Anmeldetag: **12.06.78**

(51) Int. Cl.$^2$: **C 07 J 71/00,A 61 K 31/58**

(30) Priorität: **14.06.77 DE 2727367**

(43) Veröffentlichungstag der Anmeldung:
**07.02.79 Bulletin 79/3**

(84) Benannte Vertragsstaaten:
**BE CH DE FR GB LU NL**

(71) Anmelder: **SCHERING Aktiengesellschaft Berlin und Bergkamen**
**Müllerstrasse 170-178**
**D-1000 Berlin 65. (DE)**

(72) Erfinder: **Laurent, Henry, Dr.**
**Glambecker Weg 21**
**D-1000 Berlin 28. (DE)**

(72) Erfinder: **Wiechert, Rudolf, Prof.**
**Petzower Strasse 8a**
**D-1000 Berlin 39. (DE)**

(72) Erfinder: **Wendt, Hans, Dr**
**Ringstrasse 3**
**D-1000 Berlin 38. (DE)**

(54) **Neue Kortikoide, Verfahren zu ihrer Herstellung und diese enthaltende pharmazeutische Präparate.**

(57) Kortikoide der allgemeinen Formel I

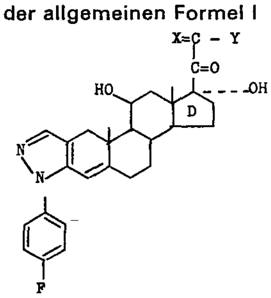

(I) .

worin
X zwei Wasserstoffatome und Y eine Hydroxyoder Alkanoyloxygruppe oder
X ein Sauerstoffatom und Y ein Wasserstoffatom, eine Hydroxy-gruppe oder eine Alkoxygruppe bedeuten, sind pharmakologisch wirksame Substanzen, die sich insbesondere zur Behandlung entzündlicher Erkrankungen eignen, werden hergestellt durch Hydrolyse von

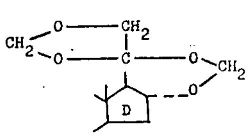

(II)

EP 0 000 471 A1

SCHERING AG
Gewerblicher Rechtsschutz
0000471

B e s c h r e i b u n g

Die Erfindung betrifft neue Kortikoide, ein Verfahren zu ihrer Herstellung und pharmazeutische Präparate, die diese Kortikoide als Wirkstoff enthalten.

Die neuen Kortikoide sind gekennzeichnet durch die allgemeine Formel I

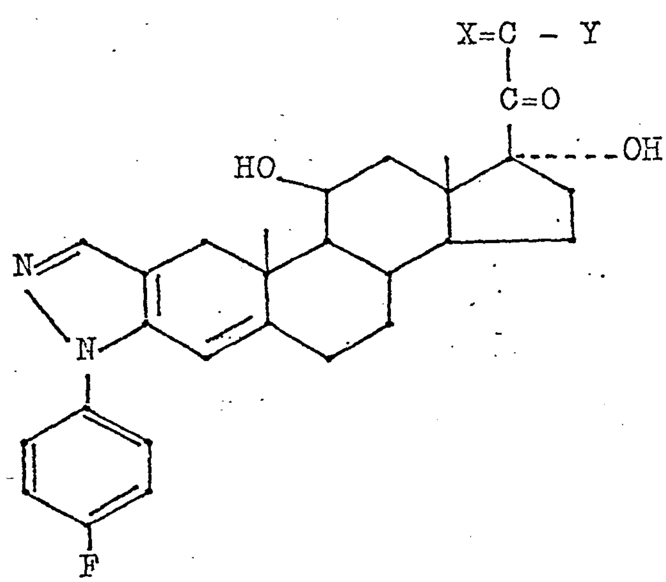

(I),

worin

X zwei Wasserstoffatome und Y eine Hydroxy- oder Alkanoyloxygruppe oder

X ein Sauerstoffatom und Y ein Wasserstoffatom, eine Hydroxygruppe oder eine Alkoxygruppe bedeuten.

SCHERING AG
Gewerblicher Rechtsschutz
0000471

Neue Kortikoide, Verfahren zu ihrer Herstellung und diese enthaltende pharmazeutische Präparate.

Vorstand: Dr. Herbert Asmis · Dr. Christian Bruhn · Hans-Jürgen Hamann
Dr. Heinz Hannse · Karl Otto Mittelsienscheid · Dr. Horst Witzel
Vorsitzender des Aufsichtsrats: Dr. Eduard v. Schwartzkoppen
Sitz der Gesellschaft: Berlin und Bergkamen

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7[illegible] 00, Bankleitzahl 100 [illegible]
Berliner Disconto-Bank AG, Berlin, Konto-Nr. [illegible], Bankleitzahl [illegible]
Berliner Handels-Gesellschaft — Frankfurter Bank [illegible]

Formular-Nr.: 1439-3

Unter einer Alkanoyloxygruppe Y soll vorzugsweise eine Gruppe verstanden werden, die sich von einer 1 bis 8 Kohlenstoffatome enthaltenden gesättigten aliphatischen Monocarbonsäure ableitet. Als Alkanoyloxygruppen seien beispielsweise genannt: Die Acetoxygruppe, die Propionyloxygruppe, die Butyryloxygruppe, die Pentanoyloxygruppe und die Hexanoyloxygruppe.

Unter einer Alkoxygruppe Y soll vorzugsweise eine 1 bis 6 Kohlenstoffatome enthaltende Alkoxygruppe, wie zum Beispiel die Methoxygruppe, Äthoxygruppe, Propyloxygruppe oder Butyloxygruppe verstanden werden.

Das erfindungsgemäße Verfahren zur Herstellung der neuen Kortikoide ist dadurch gekennzeichnet, daß man in an sich bekannter Weise die Methylendioxygruppen einer Verbindung der allgemeinen Formel II

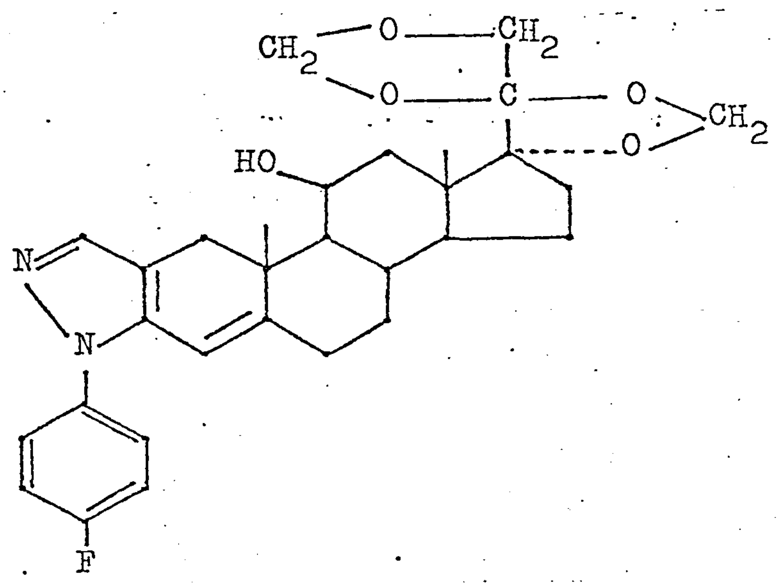

(II),

hydrolytisch abspaltet,

gegebenenfalls die 21-Hydroxygruppe verestert oder zur Aldehyd-



Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen ·
Sitz der Gesellschaft: Berlin und Bergkamen
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7005 00, Bankleitzahl 100 ...
Berliner Disconto-Bank AG, Berlin, Konto-Nr. 241/5000, Bankleitzahl 100 700 ...
Berliner Handels-Gesellschaft — Frankfurter Bank —, Berlin.

SCHERING AG
Gewerblicher Rechtsschutz
0000471

gruppe oxydiert und den erhaltenen Aldehyd in Gegenwart eines Alkohols mit Luftsauerstoff oder Mangan(IV)-oxyd oxydiert.

Die Abspaltung der Methylendioxygruppen und die Acylierung des entstandenen 21-Hydroxysteroids kann beispielsweise unter den Bedingungen durchgeführt werden, wie sie in der deutschen Patentschrift 1 072 622 beschrieben sind. Zur Überführung des 21-Hydroxysteroids in den 21-Aldehyd und die 21-Säureester sind beispielsweise die Reaktionsbedingungen geeignet, wie sie in der deutschen Offenlegungsschrift 24 41 284 beschrieben sind.

Die neuen Kortikoide der allgemeinen Formel I sind pharmakologisch wirksame Substanzen, die sich insbesondere dadurch auszeichnen, daß sie bei topischer Anwendung eine ausgeprägte antiinflammatorische Wirksamkeit besitzen, während sie systemisch gering wirksam oder unwirksam sind. Darüberhinaus zeichnen sich diese Verbindungen oft durch einen raschen Wirkungsbeginn, eine hohe Wirkungsintensität und eine lange Wirkungsdauer aus, sie haben eine günstige Resorbierbarkeit und in galenischen Zubereitungen eine relativ gute Stabilität.

Die neuen Verbindungen eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.



Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7900 00 Bankleitzahl 100 400 00
Berliner Disconto-Bank AG, Berlin, Konto-Nr. 241/5008, Bankleitzahl 100 700 00

SCHERING AG
Gewerblicher Rechtsschutz
0000471

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,01% bis 1% verwendet.

Darüberhinaus sind die neuen Verbindungen gegebenenfalls in Kombination mit den üblichen Trägermitteln und Hilfsstoffe auch gut zur Herstellung von Inhalationsmitteln geeignet.

Die nachfolgenden Beispiele dienen zur Erläuterung der Erfindung.

- 5 -

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0051

Postanschrift: SCHERING AG · D-1 Berlin 65 · Postfach [illegible]
Postscheck-Konto: Berlin-West 1175-101 [illegible]
Berliner Commerzbank AG, Berlin, Konto-Nr. [illegible]
Berliner Disconto-Bank AG, Berlin, Konto-Nr. [illegible]
Berliner Handels-Gesellschaft — Frankfurter Bank — Berlin
Konto-Nr. 14-262, Bankleitzahl 100 202 [illegible]

Formular-Nr.: 1423-2

SCHERING AG
Gewerblicher Rechtsschutz

0000471

Beispiel 1

a) Eine Lösung von 5,0 g 11ß-Hydroxy-2-hydroxymethylen-17α,20;20-21-bismethylendioxy-4-pregnen-3-on in 50 ml konzentrierter Essigsäure wird mit 1,0 g Natriumacetat und 2,4 g 4-Fluorphenylhydrazin-hydrochlorid versetzt und 5 Stunden bei Raumtemperatur gerührt. Das Reaktionsgemisch wird verdünnt und mit Dichlormethan extrahiert. Die organische Phase wird neutralgewaschen, getrocknet und im Vakuum eingeengt. Der Rückstand wird an Kieselgel chromatographiert. Mit Pentan-Äther (1:4) erhält man 1,80 g 2'-(4-Fluorphenyl)-17α,20; 20,21-bismethylendioxy-2H'-2,4-pregnadieno[3,2-c]pyrazol-11ß-ol vom Schmelzpunkt 176° C.

b) 3,5 g des vorstehend erhaltenen Produkts werden 3 Minuten mit 20 ml 37 %iger Salzsäure bei Raumtemperatur gerührt. Das Gemisch wird unter Eiskühlung in 100 ml 25 %iger Ammoniumhydroxydlösung eingegossen, der ausgefällte Niederschlag wird isoliert und an Kieselgel chromatographiert. Mit 37 - 48 % Aceton-Hexan erhält man 901 mg 2'-(4-Fluorphenyl)-11ß,17α,21-trihydroxy-2H'-2,4-pregnadieno-[3,2-c]pyrazol-20-on vom Schmelzpunkt 224° C.

Beispiel 2

Eine Lösung von 1,7 g 2'-(4-Fluor-phenyl)-11ß,17α,21-trihydroxy-2H'-2,4-pregnadieno[3,2-c]pyrazol-20-on in 40 ml Methanol wird mit 800 mg Kupfer(II)-acetat versetzt und 4 Stunden bei Raumtemperatur gerührt. Das Reaktionsgemisch wird mit Dichlormethan ver-

- 6 -

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 700 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin.

SCHERING AG
Gewerblicher Rechtsschutz
0000471

setzt und mit verdünnter Ammoniumhydroxydlösung extrahiert. Die organische Phase wird mit Wasser gewaschen, über Natriumsulfat getrocknet und im Vakuum eingedampft. Der Rückstand besteht aus 1,8 g 2'-(4-Fluor-phenyl)-11ß,17α-dihydroxy-20-oxo-2H'-2,4-pregnadieno/3,2-c/pyrazol-21-al.

Beispiel 3

1,3 g des so hergestellten Aldehyds löst man in einem Gemisch aus 15 ml Dimethylformamid und 10 ml Methanol und versetzt nacheinander mit 4,5 g aktivem Mangan(IV)-oxid, 2,1 ml konzentrierter Essigsäure und 455 mg Kaliumcyanid. Die Reaktionsmischung wird 10 Minuten bei Raumtemperatur stark gerührt und anschließend über eine Glasfilternutsche abgesaugt. Das Filtrat wird in Eiswasser eingerührt. Der entstandene Niederschlag wird mit Dichlormethan isoliert und an Kieselgel mit Aceton-Hexan chromatographiert. Man erhält 251 mg 2'-(4-Fluor-phenyl)-11ß,17α-dihydroxy-20-oxo-2H'-2,4-pregnadieno/3,2-c/pyrazol-21-säure-methylester. Schmelzpunkt 192° C (umkristallisiert aus Aceton-Hexan).

Beispiel 4

1,2 g 2'-(4-Fluor-phenyl)11ß,17α-dihydroxy-20-oxo-2H'-2,4-pregnadieno/3,2-c/pyrazol-21-al werden unter den im Beispiel 1b beschriebenen Bedingungen, jedoch mit Butanol anstelle von Methanol in 2'-(4-Fluor-phenyl)-11ß,17α-dihydroxy-20-oxo-2H'-2,4-pregnadieno/3,2-c/pyrazol-21-säure-butylester. Schmelzpunkt 150° C (umkristallisiert aus Aceton-Hexan).



Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Postanschrift: SCHERING AG · D-1 Berlin 65 · Postfach 65 03 11
Postscheck-Konto: Berlin-West 1175-101

SCHERING AG
Gewerblicher Rechtsschutz
0000471

## Patentansprüche

1. Kortikoide der allgemeinen Formel I

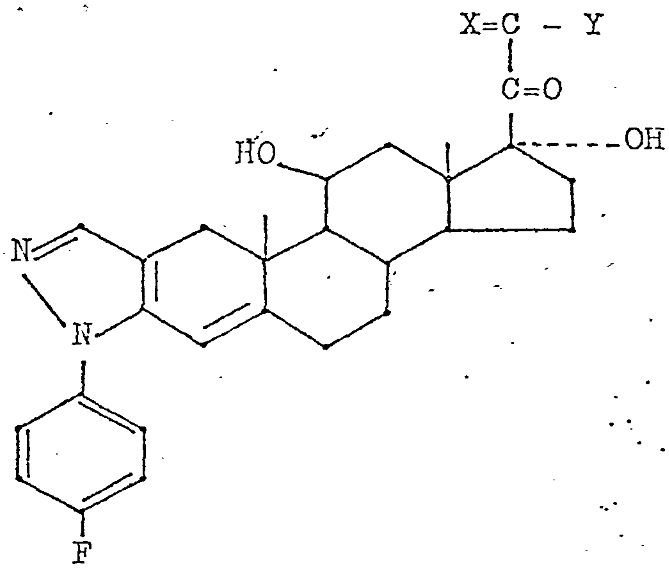

(I),

worin

X zwei Wasserstoffatome und Y eine Hydroxy- oder Alkanoyloxygruppe oder

X ein Sauerstoffatom und Y ein Wasserstoffatom, eine Hydroxygruppe oder eine Alkoxygruppe bedeuten.

2. 2'-(4-Fluorphenyl)-11ß,17α,21-trihydroxy-2H'-2,4-pregnadieno-[3,2-c]pyrazol-20-on.
3. 2'-(4-Fluor-phenyl)-11ß,17α-dihydroxy-20-oxo-2H'-2,4-pregnadieno[3,2-c]pyrazol-21-al.
4. 2'-(4-Fluor-phenyl)-11ß,17α-dihydroxy-20-oxo-2H'-2,4-pregnadieno[3,2-c]pyrazol-21-säure-methylester.



Vorstand: Dr. Herbert Asmis · Dr. Christoph Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
HR B 283 u. AG Kamen HRB 0001

Postanschrift: SCHERING AG · D-1 Berlin 65 · Postfach 65 03 11
Postscheck-Konto: Berlin West 1175-101, Bankleitzahl 100 100 10
Berliner Commerzbank AG, Berlin, Konto-Nr. 108 7005 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

5. 2'-(4-Fluor-phenyl)-11ß,17α-dihydroxy-20-oxo-2H'-2,4-pregna-dieno[3,2-c]pyrazol-21-säure-butylester.

6. Pharmazeutische Präparate, gekennzeichnet durch einen Gehalt eines oder mehrerer Kortikoide gemäß Anspruch 1 bis 5 als Wirkstoff.

7. Methode zur Behandlung von Entzündungen, dadurch gekennzeichn daß man den Patienten ein pharmazeutisches Präparat gemäß Anspruch 6 verabfolgt.

8. Verfahren zur Herstellung von Kortikoiden der allgemeinen Formel I

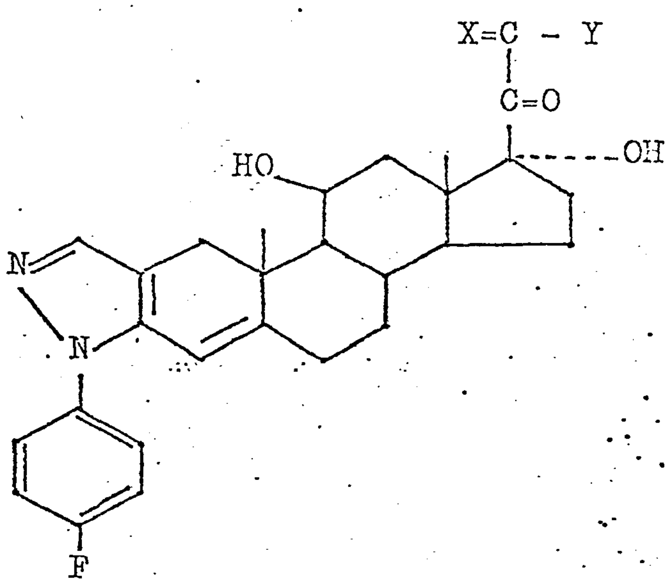

(I),

worin

X zwei Wasserstoffatome und Y eine Hydroxy- oder Alkanoyloxygruppe oder

X ein Sauerstoffatom und Y ein Wasserstoffatom, eine Hydroxy-gruppe oder eine Alkoxygruppe bedeuten, dadurch gekennzei



Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7600 00, Bankleitzahl
Berliner Disconto-Bank AG, Berlin, Konto-Nr. 241/5008, Bankleitzahl
Berliner Handels-Gesellschaft — Frankfurter Bank —, Berlin,
Konto-Nr. 14-362, Bankleitzahl 100 202 00

Formular-Nr.: 1439-2

net, daß man in an sich bekannter Weise die Methylendioxygruppen einer Verbindung der allgemeinen Formel II

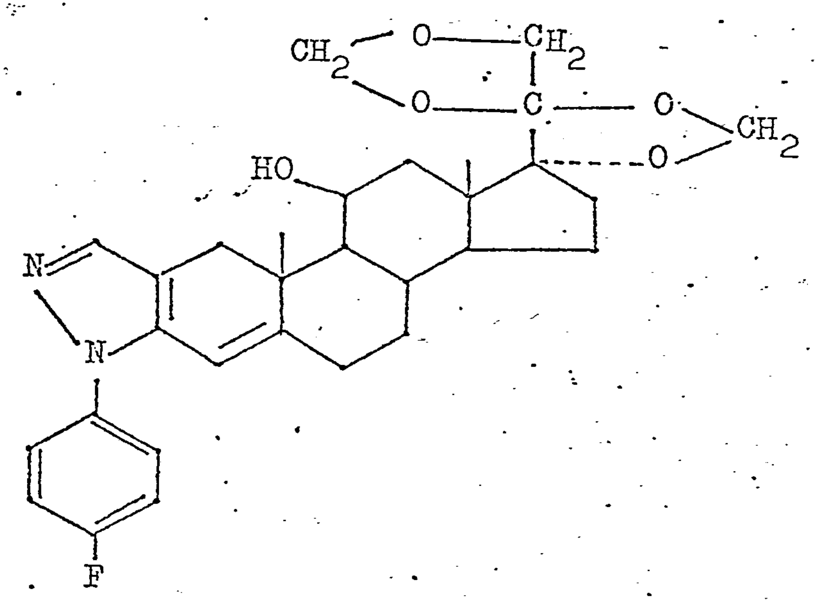

(II),

hydrolytisch abspaltet,

gegebenenfalls die 21-Hydroxygruppe verestert oder zur Aldehydgruppe oxydiert und den erhaltenen Aldehyd in Gegenwart eines Alkohols mit Luftsauerstoff oder Mangan(IV)-oxyd oxydiert.

Europäisches Patentamt

**EUROPÄISCHER RECHERCHENBERICHT**

0000471

Nummer der Anmeldung

EP 78 10 0135

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CHEMICAL ABSTRACTS, vol. 67 (1967) 107174v & J.Pharm.Pharmacol 19(9) 590-5, (1967) & CHEMICAL ABSTRACTS, 8th Collective Index Formulas S10035 f<br>* Zusammenfassung; Formula $C_{28}H_{33}FN_2O_4$ *<br>-- | 1,2,6,7 |
| | US - A - 3 704 295 (RAYMOND O.CLINTON)<br>* Ansprüche 1,2 *<br>-- | 1 |
| | DE - A - 1 418 994 (MERCK AND CO)<br>* Ansprüche 1,11 *<br>-- | 1,6 |
| | JOURNAL OF THE AMERICAN CHEMICAL SOCIETY Band 86 (1964) Seite 1520 bis 1527<br>* Seite 1520,1521,1523 *<br>---- | 1,6 |

KLASSIFIKATION DER ANMELDUNG (Int. Cl.²)

C 07 J 71/00
A 61 K 31/58

RECHERCHIERTE SACHGEBIETE (Int. Cl.²)

C 07 J 71/00
A 61 K 31/58

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| | | |